(19) Europäisches Patentamt
European Patent Office
Office européen des brevets

(11) Publication number: **0 603 412 A1**

# (12) EUROPEAN PATENT APPLICATION published in accordance with Art. 158(3) EPC

(21) Application number: **93913664.4**

(51) Int. Cl.[5]: **C07F 15/00**, A61K 31/295

(22) Date of filing: **12.05.93**

(86) International application number:
**PCT/RU93/00108**

(87) International publication number:
**WO 93/23411 (25.11.93 93/28)**

(30) Priority: **12.05.92 RU 5047273**

(43) Date of publication of application:
**29.06.94 Bulletin 94/26**

(84) Designated Contracting States:
**AT BE CH DE DK ES FR GB GR IE IT LI LU MC NL PT SE**

(71) Applicant: **EFIMENKO, Inessa Alexandrovna**
**ul. Udaltsova, 28-27**
**Moscow, 117454(RU)**
Applicant: **KURBAKOVA, Anna Prokhorovna**
**ul. Akademika Vargi, 2-193**
**Moscow 117133(RU)**
Applicant: **PONTICELLI, Gustavo**
**Via Tuveri, 12**
**I-09129 Cagliari(IT)**
Applicant: **GRAP, Sergei Romanovich**
**ul. Krasny Kazanets, 3-2-49**
**Moscow 111395(RU)**
Applicant: **IVANOVA, Nina Alexandrovna**
**ul. Nakhimova, 6-90**
**Moskovskaya obl. Khimki, 141400(RU)**
Applicant: **TRESCHALIN, Ivan Dmitrievich**
**ul. Millionschikova, 18-394**
**Moscow 115446(RU)**
Applicant: **BODYAGIN, Dmitry Alexandrovich**
**Kolomenskaya naberezhnaya, 6-89**
**Moscow, 115142(RU)**
Applicant: **KRIMKER, Vladimir Mikhailovich**
**ul. Zolotorozhskyval, 2-39**
**Moscow, 109033(RU)**
Applicant: **REVAZOVA, Julia Anatolievna**
**ul. 2 Pugachevskaya, 3-1-233**
**Moscow, 107553(RU)**

(72) Inventor: **EFIMENKO, Inessa Alexandrovna**
**ul. Udaltsova, 28-27**
**Moscow, 117454(RU)**
Inventor: **KURBAKOVA, Anna Prokhorovna**
**ul. Akademika Vargi, 2-193**
**Moscow 117133(RU)**
Inventor: **PONTICELLI, Gustavo**
**Via Tuveri, 12**
**I-09129 Cagliari(IT)**
Inventor: **GRAP, Sergei Romanovich**
**ul. Krasny Kazanets, 3-2-49**
**Moscow 111395(RU)**
Inventor: **IVANOVA, Nina Alexandrovna**
**ul. Nakhimova, 6-90**
**Moskovskaya obl. Khimki, 141400(RU)**
Inventor: **TRESCHALIN, Ivan Dmitrievich**
**ul. Millionschikova, 18-394**
**Moscow 115446(RU)**
Inventor: **BODYAGIN, Dmitry Alexandrovich**
**Kolomenskaya naberezhnaya, 6-89**
**Moscow, 115142(RU)**
Inventor: **KRIMKER, Vladimir Mikhailovich**
**ul. Zolotorozhskyval, 2-39**
**Moscow, 109033(RU)**
Inventor: **REVAZOVA, Julia Anatolievna**
**ul. 2 Pugachevskaya, 3-1-233**
**Moscow, 107553(RU)**

(74) Representative: **Ruffles, Graham Keith et al**
**MARKS & CLERK,**
**57-60 Lincoln's Inn Fields**
**London WC2A 3LS (GB)**

## (54) COMPOUNDS BASED ON PALLADIUM AND ON DERIVATIVES OF AROMATIC AMINES AND METHOD



Rank Xerox (UK) Business Services
(3.10/3.09/3.3.4)

**OF OBTAINING THEM.**

(57) New compounds are proposed based on palladium and on derivatives of aromatic amines of the following general formula (I): $[NH_2R_1R_2]_2PdCl_4$ in which $R_1$ is a group with formula (II) where Y = H; OH; $NO_2$; $X_1$ = H; OH; $X_2$ = OH; $CH_3$; $X_3$ = H; $CH_3$; $CH_2OH$; and $R_2$ = $CH_3$; $C_2H_5$, having radiomodifying activity and capable of being used in medicine. The compounds are effective to be introduced in the organism both before and after gamma radiation, as well as at chronic radiations with small doses. A method of obtaining the claimed compounds is also proposed.

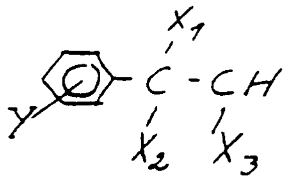 (II)

## TECHNICAL FIELD

The present invention relates to organic chemistry and medicine and more particularly to the compounds of Palladium with aromatic amine derivatives and a process for the preparation thereof. These compounds may be useful in radiative medicine for preventing mammalian species from ionizing radiation by administering to a mammal the aforesaid compounds both pre- and post-exposing to rays as well as under chronic low-dose irradiation.

## PRIOR ART

The results of doing away with the posteffect of an accident on Chernobyl atomic power plant as well as the other radiative emergencies in different countries have clearly demonstrated a limited number of the biological active compounds having a radiomodifying activity.

Known in the art are sulfur-containing radioprotective agents such as indolylalkylamines and cyanides having effect on target-cells and radioprotectors decreasing a radiation damage to a body at the expense of hemic tissue or circulatory hypoxia [Theses of Reports on the 1st congress of Radiobiology, Moscow, August 21-27, 1989, v.I, Puschino, 1989 (V.G.Vladimirov, A.G.Sverdlov. Sovremennye predstavlenia o mekhanizme radizaschitnogo effekta.-Modern Views to Radioprotective Mechanism, p.5)] [1].

However, all of the known radioprotective agents have the essential drawbacks. Drug preparations thereof are of high toxicity and do not make it possible to obtain a high enough radioprotective effect which is directly related to the dosage. Moreover, high toxicity rate rules out the possibility of their frequent administration. A peculiarity of up-to-date radioprotective agents lies in the appreciable reduction in their protective effect under multiple dose fractionation and in its practically full absence under low-dose irradiation.

Substantial peculiarity of said radioprotectors lies also in the fact that the protective effect manifests itself only when administering these compounds prior to irradiation. It makes impossible their application for the treatment of diseases caused by the radiation damage in a body.

Nowadays in connection with the current situation (accident on Chernobyl atomic power plant and certain another occasions) the alternative methods of treating a radiation damage are under intensive development. For this purpose proposed is a combination of vitamins and microelements which generally are the substances of plant origin and the other biologically active compounds [1].

Drug preparations comprising these substances operate through a body and promote post-radiation recovery processes of irradiated and damaged tissues. However, these preparations exhibit rather weak therapeutic effect [Theses of Reports on the 1st Congress of Radiobiology, Moscow, August 21-27, 1989, v.I, Puschino, 1989 (T.V.Ponamareva et al. Novye sredstva dlja selektivnoi protivoradiatsionnoi zaschity kishechnika.- Novel Means for Selective Radioprotection of Bowels, p.756-757)] [2].

According to the present invention proposed as radiomodifying agents are the novel palladium complexes with aromatic amine derivatives.

Known in the art are palladium complexes with amines (including aromatic ones) in hydrochloric acid solutions [M.A.Meretukov. Protsessy zhidkostnoi ekstraktsii v tsvetnoi metallurgii, Metallurgia (Moskva).- Solvent Extraction in Non-Ferrous Metallurgy, Metallurgy Publishers (Moscow), 1985, p.182].

These complexes are formed under separation and selective recovery of palladium and platinum group netals with amines of different chemical structure. The use of said palladium complexes as radiomodifiers had not hitherto been reported.

## DISCLOSURE OF THE INVENTION

It is an object of the present invention to provide novel compounds having a radiomodifying activity when administering them to a body both pre- and post-exposing to rays as well as under chronic low-dose irradiation conditions.

A further object of the present invention is to provide a process for the preparation of said novel compounds.

This object as stated is attained by providing novel compounds of palladium with aromatic amine derivatives of the formula I

$[NH_2R_1R_2]_2[PdCl_4]$ (I)

wherein $R_1$ represents a group of the formula

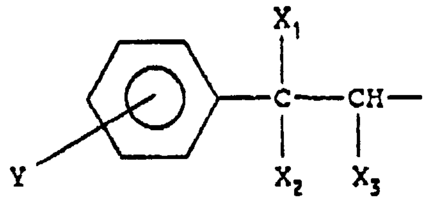

in which Y is hydrogen, hydroxy or nitro; $X_1$ is hydrogen or hydroxy; $X_2$ is hydroxy or methyl; $X_3$ is hydrogen, methyl or hydroxymethyl and $R_2$ is methyl or ethyl.

The compounds of the invention have desirable radiomodifying activity when administering to a body both pre- and post-exposing to rays as well as under chronic low-dose radiation.

In so doing these compounds provide a therapeutic effect at the dosage of lens than a maximal permissible dose (M.P.D.) by a factor of 10.

Moreover, the inventive compounds are of low toxic and in therapeutic doses fail to exhibit any side effects such as allergenicity, teratogenecity, undesirable effect on a kidney and so on.

The inventive compounds are of pyrogen-free, have bactericidal activity against come bacteria and microbes.

The compounds of the present invention can be useful in human and veterinary medicine as parenteral injections administered by intramuscular, intraperitoneal or intravenous routes and also be conventionally prepared in dosage unit form in ampules or other unite-dose or multiple-dose packages.

Therapeutically and prophylactically the instant compounds are usually given as drug preparations comprising an effective amount of an active ingredient in combination with one or more pharmaceutically acceptable carrier or diluent.

An active ingredient may be produced in the form of sterile powder for subsequent dilution in suitable vehicle for injections.

Such drug preparations having a desired clarity, stability and adaptation for parenteral use may be obtained by dissolving from 0.1 to 1.0% by weight of an active inventive compound, preferably from o.10 to 0.25% by weight in water for injections or vehicle, particularly, in isotonic medium representing the 0.9% solution of sodium chloride.

When a drug preparation is in dosage unit formulation, i.e., when a discrete unit contains a predetermined amount of an active ingredient corresponding to a fraction or multiple of the dose which is calculated to produce the desired therapeutic response, a daily dose is administered in amount of 2.5 to 80 mg/kg of a body weight, preferably from 10 to 20 mg/kg.

The compounds of the formula I are yellowish brown, odorless, nonhygroscopic crystalline substances, soluble in water and isotonic medium. When heated over 100-120 °C these compounds begin to decompose without melting; they are photostable both in the solid form and as a solution and show acid nature in isotonic medium. Specifically, a solution containing 25 g of Ephazole (compound 1) in 100 g of isotonic medium has pH from 3.6 to 4.1 within 15 minutes of its preparing.

At 25-35 °C the inventive compounds keep constant their physico-chemical properties and biological activity over 3 years.

The structure of the inventive compounds of the formula I is confirmed by IR spectra and X-ray analysis.

For a structure of the inventive compounds to be studied in detail chosen was Ephazole as a supported Example.

Conclusions regarding its chemical structure have been drawn from IR spectra for Ephazole and free ligand in the absorption range from 100 to 4000 $cm^{-1}$ measured by IR Fourier Spectrometer Bruker-IFS-113V and specified by X-ray data obtained for monocrystalline ephazole using automatic 4-circle X-ray diffractometer Syntex $P2_1$.

Moreover, a further embodiment of the present invention is a process for the preparation of the compound of the formula I comprising reacting a compound of the formula II

$[NH_2R_1R_2]Cl$ (II)

wherein $R_1$ and $R_2$ are as defined above, with palladium dichloride at a stoichiometric ratio of the components and at a temperature of 30-80 °C followed by isolation of the final product.

The isolation step is recommended to carry out in the temperature range from 30 to 80 °C.

**PREFERRED EMBODIMENT**

Among the compounds of the formula I

$[NH_2R_1R_2]_2[PdCl_4]$

wherein $R_1$ is a group of the formula

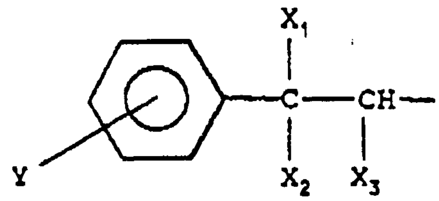

the following compounds were produced and studied according to the radical definitions listed below.

1. N-methyl-N-(1-phenyl-1-hydroxy-prop-2-yl)ammonium palladium tetrachloride (Ephazole).
   $Y = H$; $X_1 = H$; $X_2 = OH$; $X_3 = R_2 = CH_3$.
2. N-methyl-N-[2-hydroxy-2(4-nitrophenyl)ethyl]ammonium palladium tetrachloride.
   $Y = n\text{-}NO_2$; $X_1 = X_3 = H$; $X_2 = OH$; $R_2 = CH_3$.
3. N-[1,3-dihydroxy-1-(4-nitrophenyl)-prop-2-yl]ammonium palladium tetrachloride.
   $Y = n\text{-}NO_2$; $Xl = R_2 = H$; $X_2 = OH$; $X_3 = CH_2OH$.
4. N-methyl-N-(2-phenyl-2-hydroxypropyl)ammonium palladium tetrachloride.
   $Y = X_3 = H$; $X_1 = OH$; $X_2 = R_2 = CH_3$.
5. N-ethyl-N-[2-hydroxy-2(4-hydroxyphenyl)ethyl]ammonium palladium tetrachloride.
   $Y = m\text{-}OH$; $X_1 = X_3 = H$; $X_2 = OH$; $R_2 = C_2H_5$.

IR spectra for Ephazole and ligand are different in the range from 1500 to 4000 $cm^{-1}$. This makes it possible to reveal the spectral changes which take place at the formation of a complex compound. Specifically, spectrum illustrates ligand IR bands at 3340, 3300, 2840, 2760, 2470, 1965, 1890, 1817 and 1595 $cm^{-1}$ characteristic of hydrochloric salt of 1-phenyl-2-methylamino-propane-1-ol having the hydrogen bond such as

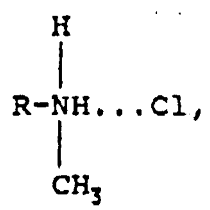

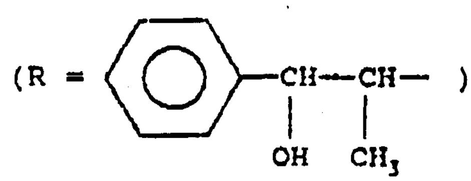

which is characterized by the absorption bands at 2470 $cm^{-1}$. Doublet 3340/3300 $cm^{-1}$ is defined by OH...Cl intramolecular hydrogen bond.

Disappearance of the above bands in IR spectrum of Ephazole brings into existence the bands applying to

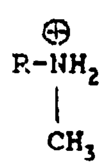

cation and having the frequencies of 2881, 2846, 2735 $cm^{-1}$(NH) as well as 1577 and 1556 $cm^{-1}(\overset{+}{N}H_2)$. Intensive doublet 3450/3400 $cm^{-1}$ applies to the valence vibration of OH group involved in the intramolecular OH...Cl hydrogen bond with chlorine atoms of acidoanion.

The availability of $[PdCl_4]^{2-}$ anion in Ephazole is confirmed my intensive IR band at 323 $cm^{-1}$ (Pd-Cl) characteristic of a plane-square anion $[PdCl_4]^{2-}$ in the compounds such as $K_2PdCl_4$ and $(NH_4)_2[PdCl_4]$.

Thus, according to IR data Ephazole is a cation-anion complex having the structure

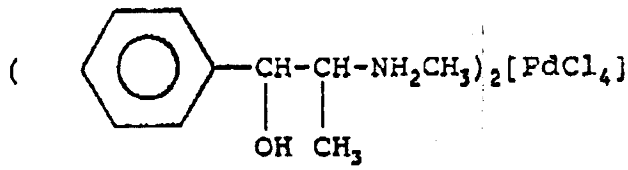

wherein two kinds of hydrogen bond occur: intramolecular hydrogen bond NH...Cl and OH...C1 as well as intermolecular hydrogen bond NH...Cl. Such a system of hydrogen bonds is well established on the basis of X-ray analysis data.

The crystal is found to construct of the anions $[PdCl_4]^{2-}$ located on the cation axis $CH_3NH_2CH(CH_3)CH$-(Phe)OH occupying a general position in the crystal. This construction as well as the geometrical characteristics of both structural units show that Pd atom has a plane-square coordination in the anion, with the ordinary bond lengths Pd-Cl being as follows: [2.272(3); 2.284(4); 2.301(1) A]. CH groups in the cation at N-C(8) positions in the atoms are in gauche conformation relative to N-C(8) band, nitrogen atom and hydroxy group are in gauche conformation relative to C(7)-C(8) band and nitrogen atom and phenyl group C(1)...C(6) are in trans-configuration relative to the same bond. Such a conformation of the cation is favorable to forming a complicated system of hydrogen bonds combining both the cations and the atoms. The cation has three "active" protons and each of them is involved in hydrogen bonds with coordinated chlorine atoms of the anions.

Hydrogen moieties in a pair of cations act as a bridge between dianions and combine them into infinite chains growing along the axes of two crystals. In so doing OH groups in each of said pair are oriented to symmetrically bonded chlorine atoms Cl(3) and Cl(3) of the same anion to form hydrogen bonds with them.

The length of the Cl(3)...H(0) bond is equal to 2.31 A with the sum of atomic van-der-Waals radiuses of 3.0 A, and bond angles in Cl(3)...H-O and in O-Cl-Pd amount to 155° and 109°, respectively. All the defined parameters conform to rather strong hydrogen bond. Two symmetric independent hydrogen bonds such as NH...Cl have a markedly different geometry. The length of the Cl(l)...H(2) bond equal to 2.19 A is shorter than that of Cl(2)...H(1)(2.40 A), bond angle in Cl(1)...H(2) amounts to 154° whereas in Cl(2)...H(1)-N it is only of 136°. These data point to the fact that the hydrogen bonds of Cl(2)...H(2) type are more strong than that of Cl(2)...H(l)-N type.

So, a molecular structure of Ephazole has been confirmed by the methods of IR and X-ray spectroscopy.

The structure of the other inventive compounds is established by IR spectroscopy data as compared to Ephazole IR spectra.

The fundamental absorption bands applying to the compounds of the present invention and their referrals are listed in Table 1.

A similarity of the compounds of the formula I in IR spectra to one another enables the conclusion to be drawn that these compounds are of the same structure, i.e. they are representatives of the cation-anion complexes.

All the compounds have two kinds of hydrogen bond: intramolecular bonds such as OH...Cl and NH...Cl and intermolecular bonds such as NH...Cl.

Furthermore, the state of Ephazole in water and 0.9% NaCl solutions as a function of its concentration and exposure time was studied by electron spectroscopy with Specord M-40 UV-Spectrometer. The studies were carried out for Ephazole solutions containing 0.05-2 wt% of the compound and at a temperature from 4 to 100°C. Electron absorption spectra were recorded immediately on addition of 0.9% NaCl solution to the weighed samples of Ephazole. From the data obtained it follows that when varying a palladium content from $3.48x10^{-3}$ to $17.40x10^{-3}$ gram-ion/l the electronic absorption spectra at the initial instant and in the range from 350 to 500 nm show a broad band at 460 nm( =153). It should be noted that in electronic spectrum of $H_2PdCl_4$ in 0.9% NaCl solution the absorption band of $[PdCl_4]^{2-}$ anion corresponds to the wavelength of 468 nm ($\epsilon$ =160). A small hypsochromic shift in the Ephazole spectrum as compared to that of $[PdCl_4]^{2-}$ anion seems to be associated with the influence of a voluminous organic cation.

Spectral characteristics for Ephazole isotonic saline solutions of various concentrations depending an the exposure time are cited in Table 2.

As follows from the Table 2 there are temporal changes in electronic spectra of Ephazole in 0.9% NaCl isotonic solutions: absorption peak shifts into a short-wave region without any change in absorption intensity of this band ($t=4h,\Delta\lambda=15nm$; $t=28h,\Delta\lambda=30nm$). The change observed is associated with the aquation of $[PdCl_4]^{2-}$ anion and equilibrium established in isotonic saline system between tetraacido form and triacidoaqua form of Ephazole:

$[PdCl_4]^{2-} + H_2O = [PdCl_3(H_2O)]^- + Cl^-$

According to spectrochemical series [1] a substitution of chlorine anion with $H_2O$ molecule is bound to result in a shift to a short-wave region. This process for producing a triacidoaqua form of Ephazole is more in evidence in the solutions with a lesser palladium concentration. The results obtained are not in contradiction with the literature data [2] regarding $[PdCl_4]^{2-}$ anion aquation. Calculations for equilibrium constants of the above system made on the basis of data cited in [2] has demonstrated that the system Ephazole/0.9% NaCl under equilibrium conditions contains 78.7% of $[PdCl_4]^{2-}$ and 21.3% of $[PdCl_3(H_2O)]^-$ anion.

A study of stability was made of the Ephazole solutions in isotonic saline medium containing 0.05; 0.1; 0.2; 0.4; 0.5; 1.0 and 2.0% of the compound at 25°C. It was discovered that solutions suitable for practical use are bound to contain from 0.05 to 0.2% of Ephazole. The time for using a 0.4% solution is not over one hour.

A stability was also Studied of Ephazole in 0.9% NaCl solutions at various temperatures equal to 4, 25, 40 and 100°C. It was shown that tested solutions could not be heated over 40°C. Lowering the temperature to 4°C stabilizes the Ephazole solutions, so it is a good practice to keep ones prepared in the cold.

Electronic absorption spectra of aqueous solutions containing 20 mg/10 ml and 40 mg/10 ml of Ephazole in the region of 350-500 nm show intensive absorption band at 424 nm ($\epsilon$ =207). After a day the spectrum undergoes appreciable modifications: absorption peak shifts to a short-wave region (411 nm) with a simultaneous marked increase in its band intensity ($\epsilon$ =247). The results observed enable the conclusion to be made that Ephazole undergoes more profound changes, viz., a hydrolysis step of $[PdCl_4]^{2-}$ anion goes to the formation of diacidoaqua species such as $[PdCl_2(H_2O]^-$ or to acidotriaqua species such as $[PdCl(H_2O)_3]^+$.

So the studies made by the method of electronic spectroscopy in respect to the state of Ephazole in water and isotonic saline solution allow a conclusion to be drawn that the water can not be used as a vehicle for drug preparations due to a profound hydrolysis observed in aqueous Ephazole solutions.

For the preparations is only to be mixed 0.9% aqueous NaCl. Isotonic saline solution stabilises the equilibrium between tetraacido form and triacidoaqua form of Ephazole.

The compounds of the present invention were tested for evaluating toxicity and biological data.

Tests were carried out on male hybrid mice $F_1(CBAxC_{57}BL)$. About two thousands animals have been used in tests.

Acute toxicity off the invention compounds was determined in experiments on mice after a single intravenous and intraperitoneal administration. Tested compounds were injected in the form of 0.1 to 1.0 wt% solutions by syringe with the entry needle. Intraperitoneal route was effected by administering a suspension of the compounds in 1% starch size using a syringe and a metal probe. The animals were observed for a month; there have been recorded the number of dead mice, time of their death, state and behavior of tested animals. Toxicity doses were calculated and statistically processed via Litchfield-Wilcoxon technique modified by Z.Rott.

Results of the acute toxicity assay are listed in table 3.

Subchronic toxicity rates of the compounds of the formula I were determined in experiments on rats and dogs under daily fivefold or tenfold intravenous administration.

It has been shown that when administered in therapeutical dosage the inventive compounds fail to influence the fundamental mechanism of cell-bounded and humoral immunity as well as have no pathological effect on a biochemical and morphological make-up of blood.

When penetrating into a paravenous cellular tissue the inventive compounds are able to cause a local tissue response similar to the effect of widely used drugs such as calcium chloride, bicarbazine etc.

When used in the interval of high and lethal doses the compounds of the present invention cause alterations in brain vasculature, in part, mydriasis, exophthalmos, short-breathing and vomiting; they can have a pathologic effect on a vascular system producing a change in the size of "R" and "S" peaks in an electrocardiogram and cause a sinus arrhythmia as well.

A total irradiation of the tested animals was effected by means of Stebel-3-A apparatus with the dose rate of 8.2 R/sec (Cesium-137 as a radiation source).

Before irradiation control group of animals was intravenously administered isotonic saline solution in amount of 0.3 ml per mouse. Tested groups was intravenously administered (in tail vein) a solution containing 0.35% of the compound of the formula I in isotonic saline medium at the dosage from 2.5 to 80 mg per 1 kg of an animal body weight.

The mice were placed into the glass containers inserted into irradiation zone. The radiation sources were arranged in the form of "squirrel cage". The radiation doses were established by duration of the stay in active zone. A dosimetric control showed that the close difference inside a container is not over 10%. The animals were irradiated in the range from 4.0 to 10 Gr.

The tested animals were observed for three days; there have been recorded a lethality rate, a time of death, state and behavior of mice subjected to irradiation. A survival rate was determined on the 8th and 30th day of observation. From the data obtained were derived the curves illustrating a dependence of the survival rate upon a radiation dosage on a percentage basis. $LD_{50/8}$ and $LD_{50/30}$ values were estimated by extrapolation to the dosage scale.

A radiomodifying effect was evaluated by a dose change factor (DCF) calculated from the ratio of $LD_{50}$ in test to $LD_{50}$ in control.

The data obtained for Ephazole are given in Tables 4-6. From 10 to 40 animals per one "point" (i.e. per one preset radiation dose from the above test range) were used in experiments.

The radiomodifying activity data for the compounds 2-5 of the present invention are given in Table 7.

A radiomodifying effect of the compounds of tile formula I manifests itself when administering them to a body both directly before and directly after irradiation with the DCF value of 1.10 and at the drug dosage of 10 mg/kg. It was revealed both from intestinal and marrowy death of the animals.

In the latter case a radiomodifying effect is beginning to emerge from the dosage of 2.5 mg/kg (DCF = 1.07) and practically flattens out at DCF = 1.1 in the dosage range of 10 to 40 mg/kg. From the rate of lethality caused by intestinal syndrome at the dosage from 2.5 to 5 mg/kg the inventive compounds have no any radiomodifying activity which is beginning to emerge from 10 mg/kg and remains constant up to 40 mg/kg. The preferred range of the dosage is from 10 to 20 mg/kg.

Using a preparation in the amount of 10 mg/kg 4 hours prior to irradiation as well as administering at the same dose within 4 and 6 hours after irradiation showed no any radiomodifying activity.

At the same time equivalent radiomodifying effect of the inventive compounds attainable both directly before irradiation and immediately after it indicates that this effect does follow irradiation.

So, on the basis of the data obtained it follows that the compounds of the formula I are not typical radioprotective agents.

Their effective use 15 min before irradiation would seem to be suggestive of radioprotective properties. However, the fact that no increase in efficacy of these compounds is observed for higher dosages under the same administering conditions does not support this assumption. It is evident that a prolonged radiomodifying effect occuring for the inventive compounds is connected with intensifying a repair of sublethal damages.

The freedom from radiomodifying activity for the compounds of the formula I when administering 4 hours before irradiation indicates that a biological efficiency of the preparations is not over this period.

Specifically, an appreciable drop in radiomodifying effect of Ephazole to DCF = 1.03 (when administering within 4 hours after irradiation) and its full absence when using Ephazole within 6 hours after irradiation also indicates that this effect is harnessed owing to intensifying the repair of sublethal damages. The freedom from radiomodifying influence when administering Ephazole 4 hours before and within 6 hours after irradiation practically excludes a realization of this effect in a body of irradiated animal by the "tissue recovery intensification" mechanism. Attempt to explain a radiomodifying activity of Ephazole by the "oxygen effect" mechanism is groundless (due to observed efficacy of the drug preparation on administering it after irradiation) and depends on the state of marrow cells and bowels.

The experiments on $LD_{50/30}$ under two-fold irradiation of mice at the dosage of 4 Gr after 1st fraction and at the dosage from 4 to 6 Gr after 2nd fraction indicate that when used within 15 min after the first fraction of irradiation the compounds of the formula I make it possible to increase a survival of mice (with DCF = 1.06) at the expense of intensifying a repair of marrow cells; at the same time using these compounds within 15 min after the first and the second fraction allows DCF value to increase up to 1.11. It occurs at the expense of a total increase in repair of sublethal damages caused by both the first and the second radiation dose.

The data as presented demonstrate an efficacy of drugs under low-dose irradiation. Taking into account a low toxicity of drugs and substantiate breadth of therapeutic effect (from 10 to 80 mg/kg with M.P.D. = 110 mg/kg), the compounds of the formula I can be successfully used under long-term (chronic) low-dose irradiation.

This assumption is supported by tests under five-fold fractionating a radiation dose using the inventive compounds at the dosage of 10 mg/kg within 15 min after each fraction. This tests disclose a possibility to increase the effectiveness of therapy employed (DCF = 1.47, Table 6).

The data obtained having analyzed, the following conclusions can be drawn.

1. The inventive compounds relate to a novel group of the substances having a radiomodifying activity.
2. A radiomodufying effect exists both in the course and after irradiation and radically differs these compounds from the known radioprotective agents efficacy of which show itself merely on administering before irradiation.
3. A functional mechanism of the inventive compounds and metabolites thereof in a body of mammals is realized by intensifying a repair of sublethal radiation damages to intestinal and marowy cells in the irradiated animals. In so doing the extent of radiomodifying effect is independent of radiation dose in the range from 4.0 to 10 Gr (survival curves in control and test are parallel to one another).
4. The essential difference of the inventive compounds from the known radioprotectors lies in the fact that radiomodifying effect remains practically constant in the range of dosages from 10.0 to 80.0 mg/kg (DCF = 1.1). This permits using low dosages of drug preparations which are far from toxicity level and moreover, makes possible a repeated usage under chronic low-dose irradiation when all the available radioprotective agents are ineffective.
5. All the distinctive features render the compounds of the formula I promising for the treatment and prophylaxis of radiation damages to mammals (including a man) since fractionated irradiation results in DCF = 1.47.
6. A therapeutic efficacy of drugs currently used in radiology drops on fractionating a radiation dose and is practically reduced to zero at the extra-low doses of irradiation.
7. An effectiveness of the inventive compounds is increased with increasing the number of irradiation fractions.

A process as claimed for the preparation of the compounds of the formula I is affected as follows.

A compound of the formula II is subjected to a reaction with palladium dichloride at the stoichiometric ratio of the starting compounds using conventional equipment.

For this purpose the weighed sample of a compound of the formula II is mixed with the weighed sample of palladium dichloride and the mixture obtained is allowed to stand up to completed reacting between starting compounds at a temperature of 30-80 °C. In so doing the temperature is to be not over 80 °C in order for the reaction to occur in a desired direction without any decomposition of the reaction products.

At a temperature less than 30 °C the reaction is not proceeded at all.

The target product is isolated from the reaction mixture as a crystalline precipitate using conventional techniques.

The product obtained as crystals is dried and analyzed by elemental analysis, IR spectroscopy and the other methods commonly used in chemical practice.

The yield of the final product amounts to 88.2-97.5% of the theoretical (on the basis of palladium introduced into reaction).

To better understand the present invention below are the following Examples for producing a compound of the formula I which arc not to be construed as limiting the invention.

Example 1

N-methyl-N-(1-phenyl-1-hydroxy-prop-2-yl)ammonium palladium tetrachloride (Ephazole).

Palladium dichloride (1.348 g, 7.77 mmol) is dissolved in water (10 ml) acidified with 10N HCl (0.2 ml), filtered into a flat-bottom flask (50 ml) to which is added 3.066 g (15.52 mmol) of 1-phenyl-2-methylaminopropanol-1 in distilled water (10 ml, 1.108 mol). The reaction mixture is heated under periodic stirring on a water bath at a temperature not over 80 °C up to completed dissolving the reagents. The solution obtained is then cooled to 40 °C, filtered firstly through a glass filter and then through a membrane filter (with 0.42 mcm porosity), evaporated to dryness at 80 °C and cooled to 25 °C resulting in a completed crystallization of the product. A crystalline substrate is dried in desiccator at 60 °C under atmospheric pressure up to a constant weight.

This afforded 4.31 g of the desired product or 97.5% of the theoretical (palladium introduced basis).

| For $C_{20}H_{32}N_2O_2PdCl_4$ | | | |
|---|---|---|---|
| Calculated, % :<br>Found, % : | Pd 18.32;<br>Pd 18.62; | Cl 24.42;<br>Cl 24.30; | N 4.83<br>N 4.49. |

Example 2

N-methyl-N-[2-hydroxy-2(4-nitrophenyl)ethyl]ammonium palladium tetrachloride.

Palladium dichloride (1.00 g, 5.64 mmol) is dissolved in water (10 ml) acidified with 10N HCl (0.2 ml) and filtered into a flat-bottom flask (50 ml). To the solution obtained is added a filtered solution containing 2.21 g (11.28 mmol) of 1-(4-nitro-phenyl)-2-methylaminoethanol in 2N HCl (10 ml). Crystalline precipitate is formed immediately. The reaction mixture is allowed to stand at 30-40 °C for 1 hour under stirring and then filtered off at a room temperature. A residue obtained is dried in desiccator at 60 °C under atmospheric pressure up to a constant weight.

This afforded 4.31 g of the desired product or 97.5% of the theoretical (palladium introduced basis).

| For $C_{18}H_{26}N_4O_6PdCl_4$ | | | |
|---|---|---|---|
| Calculated, % :<br>Found, % : | Pd 16.56;<br>Pd 16.54; | Cl 22.06;<br>Cl 22.12; | N 8.12<br>N 8.28. |

Example 3

N-[1,3-dihydroxy-1-(4-nitrophenyl)-prop-2-yl]ammoniumpalladium tetrachloride.

Palladium dichloride (1.13 g, 6.35 mmol) is dissolved in water (10 ml) acidified with 10N HCl (0.2 ml) and filtered into a flat-bottom flask (50 ml). To this solution is added a filtered solution containing 3.16 g (12.7 mmol) of 1-(4-nitro-phenyl)-2-amino-propanol-1,3-diol hydrochloride in water (20 ml). The reaction mixture is evaporated under periodic stirring using a water bath at a temperature of 80 °C to dryness. To a residue is added a water (25 ml) and the mixture is evaporated to a minimal volume (this procedure is twice repeated). A crystalline substance obtained after cooling is dried in a desiccator at 60 °C under atmospheric pressure to constant weight.

This afforded 4.00 g of the desired product or 93.5% of the theoretical (palladium introduced basis).

| For $C_{18}H_{26}N_4O_8PdCl_4$ | | | |
|---|---|---|---|
| Calculated, % :<br>Found, % : | Pd 15.77;<br>Pd 15.96; | Cl 21.02;<br>Cl 20.97; | N 8.30<br>N 8.37. |

Example 4

N-methyl-N-(2-phenyl-2-hydroxypropyl)ammonium palladium tetrachloride.

Palladium dichloride (0.44 g, 2.5 mmol) is dissolved in water (30 ml) acidified with 10N HCl (0.2 ml) and filtered into a flat-bottom flask (100 ml). To this solution is added a filtered solution containing 1.07 g (5 mmol) of 2-phenyl-1-methylamino-propane-2-ol hydrochloride in water (20 ml). The reaction mixture is evaporated under periodic stirring using a water bath at a temperature of 60 °C to a minimal volume. A crystalline substance obtained after cooling is filtered and dried in a desiccator at 60 °C under atmospheric pressure to constant weight.

This afforded 1.35 g of the desired product or 93% of the theoretical (palladium introduced basis).

| For $C_{20}H_{32}N_2O_2PdCl_4$ | | | |
|---|---|---|---|
| Calculated, % :<br>Found, % : | Pd 18.32;<br>Pd 18.58; | Cl 24.42;<br>Cl 24.83; | N 4.83<br>N 4.82. |

Example 5

N-ethyl-N-[2-hydroxy-2(4-hydroxyphenyl)ethyl]ammoniumpalladium tetrachloride.

Palladium dichloride (0.99 g, 5.35 mmol) is dissolved in water (30 ml) acidified with 10N HCl (0.2 ml) and filtered into a flat-bottom flask (100 ml). To this solution is added a filtered solution containing 2.42 g (11.1 mmol) of 2-(3-hydroxyphenyl)-2-ethylaminoethanol hydrochloride in water (20 ml). The reaction mixture is evaporated under periodic stirring using a water bath at a temperature of 60°C to a minimal volume. To a residue is added a water (25 ml) and the mixture is evaporated to dryness (this procedure is twice repeated). A thick precipitate obtained after cooling is dried in a desiccator at 60°C under atmospheric pressure to constant weight.

This afforded 3.00 g of the desired product or 88.2% of the theoretical (palladium introduced basis).

| For $C_{20}H_{32}N_2O_4PdCl_4$ | | | |
|---|---|---|---|
| Calculated, % :<br>Found, % : | Pd 17.37;<br>Pd 18.24; | Cl 23.15;<br>Cl 24.56; | N 4.57<br>N 4.90. |

Example 6 (preparation of drug)

A. For animals

Ephazole (0.05 g) as a sterile substance is placed to a bottle to which is added sterile isotonic saline solution for injections (20 ml). The bottle is then shaken up to a completed dissolving the solids. A solution prepared is intravenously administered immediately after preparation at the rate of no more than 1 ml/sec.

B. For a man

To a bottle containing Epahzole (0.05 g) as a sterile substance and sterile sodium chloride (0.180 g) is added a distilled water for injections (20 ml). The bottle is shaken up to a completed dissolving the solids. The solution prepared is intravenously administered immediately after preparation.

**INDUSTRIAL USAGE**

The compounds of the formula I can be used as radiomodifying agents for the treatment and prophylaxis of radiation damages to mammals including a man.

Table 1

Particular fundamental frequencies ($cm^{-1}$) observed in IR spectra for Ephazole and its analogs

| 1-phenyl-2-methyl-propane-1-ol-hydrochloride | Compound 1 Ephazole | Compound 2 | Compound 3 | Compound 4 | Compound 5 |
|---|---|---|---|---|---|
| 3340<br>OH...Cl<br>3300 | 3450<br>OH...Cl<br>3398 | 3404<br>OH...Cl<br>3380 | 3450<br>OH...Cl<br>3390 | 3460<br>OH...Cl<br>3408 | 3504 OH not<br>3440 inv.in<br>3380 H-bond<br>OH...Cl |
| 3085<br>CH(Phe)<br>3030 | 3036<br>CH(Phe)<br>3032 | 3200<br>3100 CH(Phe)<br>3076 | 3292<br>3215<br>3150 CH(Phe)<br>3108<br>3036 | 3172<br>3150 CH(Phe)<br>3060<br>3028 | 3140<br>3080 CH(Phe)<br>3036 |
| 2960<br>2930 $CH(CH_3)$<br>2860<br>2840<br>2760<br>2470 NH...Cl<br>1605<br>CH(Phe)<br>1595 $NH_2$ | 2936 $CH(CH_3)$<br>2965 $CH(CH_3)$<br>2881<br>2846 NH...Cl<br>2735<br>1602 CHPhe<br>1577 $NH_2$<br>1556 $NH_2$ | 2965<br>2935 $CH(CH_3)$<br>2900<br>2860<br>1608 CH(Phe)<br>1596<br>1565 $NH_2$ | 2984<br>2940 $CH(CH_3)$<br>2920<br>2880<br>1604 CH(Phe)<br>1596<br>1570 $NH_2$ | 2972<br>2960 $CH(CH_3)$<br>2925<br>2860<br>1604<br>1590 CH(Phe)<br>1562 NH | 2985<br>2970<br>2960<br>2950 $CH(CH_3)$<br>2935<br>2860<br>1602<br>1590 CH(Phe)<br>1566 NH |
| 525<br>452<br>337<br>295 | 520<br>503<br>441 Pd-Cl<br>323 | 323 Pd-Cl | 329 Pd-Cl | 328 Pd-Cl | 324 Pd-Cl |

Table 2

Spectral characteristics for Ephazole solution of different concentration in isotonic saline medium as a function of the exposure time

| Ephazole, mg/10ml | % | $Cx10^3$ gram-ion/l | Exposure time, h | Wavelength, nm | ε |
|---|---|---|---|---|---|
| 20 | 0.2 | 3.48 | | | 151.7 |
| 30 | 0.3 | 5.22 | | | 153.2 |
| 40 | 0.4 | 6.95 | | | 153.1 |
| 50 | 0.5 | 8.69 | | | 156.5 |
| 60 | 0.6 | 10.4 | 0 | 460 | 156.7 |
| 70 | 0.7 | 12.2 | | | 155.7 |
| 80 | 0.8 | 13.9 | | | 157.6 |
| 90 | 0.9 | 15.6 | | | 157.0 |
| 100 | 1.0 | 17.4 | | | 154.6 |
| 20 | 0.2 | 3.48 | | 445 | 145.0 |
| 50 | 0.5 | 8.69 | | 447 | 154.7 |
| 70 | 0.7 | 12.20 | 4 | 450 | 155.4 |
| 90 | 0.9 | 15.60 | | 452 | 156.0 |
| 100 | 1.0 | 17.40 | | 456 | 153.7 |
| 20 | 0.2 | 3.48 | | 430 | 163.2 |
| 50 | 0.5 | 8.69 | | 437 | 157.4 |
| 70 | 0.7 | 12.20 | 28 | 445 | 156.0 |
| 90 | 0.9 | 15.60 | | 448 | 156.8 |
| 100 | 1.0 | 17.40 | | 450 | 154.7 |

Table 3

Acute toxicity data for the compounds of the formula I on male mice $F_1(CBA_{57}BL)$

| Compound (Example No.) | Administration route | $LD_{50}$ mg/kg | $LD_{10}$ mg/kg |
|---|---|---|---|
| Ephazole | i.v. | 140(125-155) | 110 |
| Ephazole | i.p. | 245(205-285) | 180 |
| 2 | i.g. | 95(107-83) | 63 |
| 3 | i.v. | 90(97.5-82.5) | 70 |
| 4 | i.v. | 100(109.5-91.5) | 75 |
| 5 | i.v. | 15(16.8-13.2) | 10 |

i.v. - intravenous; i.g. - intragastral; i.p. - intraperitoneal

Table 4

Radiomodifying effect of Ephazole under total γ-irradiation of mice with intravenous administration

| Preparation dosage, mg/kg | Administering time before irradiation, min | Administering time after irradiation, min | $LD_{50/8}$ (Gr) | DCF based on $LD_{50/8}$ | $LD_{50/30}$ (Gr) | DCF based on $LD_{50/30}$ |
|---|---|---|---|---|---|---|
| 2.5 | 15 | - | 8.0 | 1.0 | 7.25 | 1.07 |
| 5.0 | 15 | - | 8.0 | 1.0 | 7.5 | 1.09 |
| 10.0* | 15 | - | 9.0 | 1.11 | 8.0 | 1.11 |
| 15.0 | 15 | - | 9.0 | 1.08 | - | - |
| 20.0 | 15 | - | 9.5 | 1.13 | 7.8 | 1.04 |
| 40.0 | 15 | - | 9.0 | 1.1 | 8.25 | 1.10 |
| 80.0 | 15 | - | - | - | 8.5 | 1.20 |
| 80.0 | - | 15 | - | - | 7.75 | 1.12 |
| 10.0 | - | 15 | 8.7 | 1.1 | 8.0 | 1.10 |
| 10.0 | 4 hours | - | 8.0 | 1.0 | 7.3 | 1.00 |
| 10.0 | - | 4 hours | 8.25 | 1.03 | 7.5 | 1.03 |
| 10.0 | - | 6 hours | 7.9 | 1.0 | 7.4 | 1.00 |
| - | - | - | 8.1 | 1.0 | 7.2 | 1.00 |

*according to experimental data

Table 5

Radiomodifying effect of Ephazole under intravenous administration at the dosage of 10 mg/kg and two-fold $\gamma$-irradiating the mice at 4 hours intervals between fractional doses

| Administering time | Radiation dose for 1st fraction (Gr) | Radiation dose for 2nd fraction (Gr) | Survival rate, % | $LD_{50/30}$ (Gr) | DCF |
|---|---|---|---|---|---|
| - | 4.0<br>4.0<br>4.0 | 4.0<br>4.5<br>5.0 | 90<br>60<br>30 | 8.7 | 1.0 |
| Within 15 min after 1st fraction | 4.0<br>4.0<br>4.0 | 4.5<br>5.0<br>5.5 | 90<br>70<br>30 | 9.25 | 1.06 |
| Within 15 min after 1st and 2st fraction | 4.0<br>4.0<br>4.0 | 5.0<br>5.5<br>6.0 | 90<br>60<br>20 | 9.6 | 1.11 |

$LD_{50/30}$ were calculated from survival curves obtained by a total dose scale for two fractions

Table 6

Radiomodifying effect of Ephazole under intraperitoneal administartion at the dosage of 10 mg/kg and five-fold $\gamma$-irradiation of mice at 24 hours intervals between fractional doses

| Dosage, mg/kg | Time after irradiation, min | Irradiation dose on a fraction, Gr | $LD_{50/30}$, Gr | DCF |
|---|---|---|---|---|
| -<br>-<br>-<br>- | -<br>-<br>-<br>- | 2.0<br>2.5<br>3.0<br>3.5 | 12.75 | 1.0 |
| 10<br>10<br>10<br>10 | 15<br>15<br>15<br>15 | 2.0<br>2.5<br>3.0<br>3.5 | 18.74 | 1.47 |

Table 7

Radiomodifying effect observed when administering the compounds 2-5

| Test conditions | Irradiation dose, Gr | Survival rate, % | $LD_{50/30}$, Gr | DCF |
|---|---|---|---|---|
| Irradiation + compound 4 (10mg/kg) after 15 min | 7.5<br>8.0<br>8.5 | 80<br>20<br>10 | 7.75 | 1.07 |
| Irradiation + compound 3 (10mg/kg) after 15 min | 7.5<br>8.0<br>8.5 | 60<br>30<br>0 | 7.7 | 1.07 |
| Irradiation + compound 5 (10mg/kg) after 15 min | 7.5<br>8.0<br>8.5 | 70<br>20<br>0 | 7.75 | 1.07 |
| Compound 5 (10mg/kg) + irradiation after 15 min | 7.5<br>8.0<br>8.5 | 90<br>70<br>30 | 8.25 | 1.14 |
| Irradiation + compound 2 (10mg/kg) after 15 min | 7.0<br>7.5<br>8.0 | 90<br>70<br>30 | 8.0 | 1.10 |
| Irradiation (control) | 7.0<br>7.5<br>8.0 | 60<br>40<br>0 | 7.25 | 1.00 |

## Claims

**1.** A compound of the formula I

$[NH_2R_1R_2]_2[PdCl_4]$ (I)

wherein $R_1$ represents a group of the formula

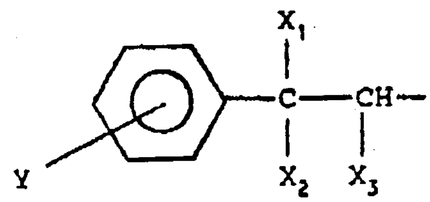

in which Y is hydrogen, hydroxy or nitro; $X_1$ is hydrogen or hydroxy; $X_2$ in hydroxy or methyl; $X_3$ is hydrogen, methyl or hydroxymethyl and $R_2$ is methyl or ethyl.

**2.** A process for the preparation of a compound of the formula I which comprises reacting a compound of the formula II

$[NH_2R_1R_2]Cl$ (II)

wherein $R_1$ and $R_2$ are as defined above, with palladium dichloride at the stoichiometric ratio of the reagents and at a temperature from 30 to 80 °C followed by isolation of the final product from the reaction mixture.

**3.** A process according to claim 2 **characterized** in that the final product is isolated from the reaction mixture at a temperature from 30 to 80 °C.

**4.** The use of a compound of the formula I as radiomodifying agents for the treatment and prophylaxis of the radiation damages to mammals including a man.

**5.** The use according to claim 4 **characterized** in that a compound of claim 1 is administered to a body of mammal both in the course of irradiation and after it.

**6.** Drug preparation comprising an effective amount of a compound of claim 1 in combination with pharmaceutically acceptable vehicle.

**7.** Drug preparation according to claim 6 characterized in that used as a vehicle is sterile isotonic solution for injections.

**8.** Drug preparation according to claim 7 characterized in that used as a vehicle is distilled water and a compound of claim 1 is previously mixed with sterile sodium chloride.

**INTERNATIONAL SEARCH REPORT**

International application No.
PCT/RU93/00108

**A. CLASSIFICATION OF SUBJECT MATTER**

$IPC^5$ : C07F 15/00; A61K 31/295

According to International Patent Classification (IPC) or to both national classification and IPC

**B. FIELDS SEARCHED**

Minimum documentation searched (classification system followed by classification symbols)

$IPC^5$ : C07F 15/00; A61K 31/295

Documentation searched other than minimum documentation to the extent that such documents are included in the fields searched

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| A | EP, A2, 0287317 (THE BRITISH COLUMBIA CANCER FOUNDATION), 19 October 1988 (19.10.88) --- | 1-8 |
| A | V. G. VLADIMIROV ET AL. "RADIOPROTEKTORY : STRUKTURA I FUNKTSII, 1989, KIEV, NAUKOVA DUMKA, pages 22, 215 - 218 --- | 1-8 |
| A | US, A, 4584316 (RESEARCH CORPORATION), 22 April 1986 (22.04.86) the abstract | 1-8 |

----------

☐ Further documents are listed in the continuation of Box C. ☐ See patent family annex.

* Special categories of cited documents:

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step when the document is taken alone

"Y" document of particular relevance; the claimed invention cannot be considered to involve an inventive step when the document is combined with one or more other such documents, such combination being obvious to a person skilled in the art

"&" document member of the same patent family

| Date of the actual completion of the international search | Date of mailing of the international search report |
|---|---|
| 30 August 1993 (30.08.93) | 22 September 1993 (22.09.93) |
| Name and mailing address of the ISA/ RU | Authorized officer |
| Facsimile No. | Telephone No. |